# EUROPÄISCHE PATENTSCHRIFT

(11) **EP 1 200 551 B1**
(45) Veröffentlichungstag und Bekanntmachung des Hinweises auf die Patenterteilung: **05.11.2003**
(21) Anmeldenummer: 97954752.8
(22) Anmeldetag: 23.12.1997
(51) Int. Cl.: C12M 1/107

(54) **BIOGASFERMENTERANLAGE**
BIOGAS FERMENTER
FERMENTEUR POUR LA PRODUCTION DE BIOGAZ

(43) Veröffentlichungstag der Anmeldung: 02.05.2002
(73) Patentinhaber: Hermann, Gerhard, 4210 Unterweitersdorf (AT); Hanel, Michael, 4040 Linz (AT)
(72) Erfinder: Hermann, Gerhard, 4210 Unterweitersdorf (AT)
(74) Vertreter: Dupal, Helmut, Dipl.-Ing.
(86) Internationale Anmeldenummer: EP9707271
(87) Internationale Veröffentlichungsnummer: WO98028402

(56) Entgegenhaltungen:
- EP-A- 0 045 114
- EP-A- 0 521 302
- WO-A-83/03884
- DE-C- 19 608 996
- US-A- 4 184 892
- US-A- 4 436 818
- US-A- 4 437 987
- US-A- 4 579 654

## Beschreibung

Die Erfindung betrifft eine Biogasfermenteranlage mit wenigstens einem Faulbehälter für eine auszufaulende Flüssigkeit, insbesonders Gülle, mit den Merkmalen des Oberbegriffes des Anspruches 1.

Solche Anlagen wurden bisher in zahlreichen Varianten gebaut und betrieben, dabei werden häufig Faulbehälter auch in den Boden eingelassen und mit einer Behälterfolie zur Abdichtung versehen, wobei der Behälter mit einer schwarz eingefärbten Folie, z.B. aus PE oder dergl., zum Auffangen des Biogases überdeckt ist. Der Faulbehälter ist dann insgesamt mit einem Schutz aus einer sehr reißfesten Folie gegen Witterungseinflüsse überdeckt, wie sie für Gewächshäuser verwendet wird.

Bei einem Faulbehälter ist es schwierig, eine wirksame Wärmedämmung zum Boden hin herzustellen und bei den bisher verwendeten Behälterformen ist es auch schwierig eine gute Entleerung der behandelten Flüssigkeit und eine gute Durchmischung während der Vergärung sicher zu stellen.

Die Herstellung eines gegen Witterung stabilen Biogasfolienbehälters mit einer ebensolchen Schutzabdeckung ist ohne zusätzlichen Bauaufwand, besonders bei größeren Anlagen, nicht mehr möglich.

Bei aufblasbaren Schutzabdeckungen, wie sie bisher angewendet werden, muß die Druckhaltung gesteuert sein um die Biogasentwicklung zu berücksichtigen, was einen erheblichen zusätzlichen Regelungsaufwand erfordert.

Steife Verkleidungen als Schutzabdeckungen sind bereits bekannt, bei denen aber ein wesentlich größerer und teurerer Konstruktionsaufwand notwendig ist.

Anlagen dieser Art sind in den Patentanmeldungen US 4,436,818 und US 4,437,987 offenbart.

Darüber hinaus ist die Herstellung eines möglichst trockenen Biogases mit möglichst hohem Wasserstoffgehalt und weitgehend herabgesetztem Gehalt an Schwefelverbindungen, bei vergleichbar geringem Aufwand, für die weitere Verwendung des Biogases von Bedeutung.

Aufgabe der Erfindung ist es, eine Biofermenteranlage mit einem Faulbehälter zu schaffen, bei dem sowohl der Behälter als auch der Gasbehälter und die Schutzabdeckung mit einem sehr einfachen Aufbau hergestellt werden können und bei dem das Befüllen und Entleeren der Flüssigkeit sowie die Behandlung des Biogases mit geringem Bau- und Wartungsaufwand möglich ist, bei möglichst einfachem Aufbau der Anlage.

Diese Aufgabe wird bei einer Biofermenteranlage mit den Merkmalen des Gattungsbegriffes des Anspruches 1 mit Hilfe der Merkmale des kennzeichnenden Teiles desselben gelöst. Die Unteransprüche betreffen besonders vorteilhafte Ausgestaltungen der Erfindung, wobei Anspruch 1 und die Unteransprüche gleichzeitig einen Teil der Beschreibung der Erfindung bilden.

Die Anwendung einer zentral angeordneten Tragsäule, die am Fundament des Faulbehälters verankert ist und die den Foliengengasbehälter und die Schutzabdeckung trägt, ermöglicht den Aufbau von sehr stabilen Anlagen auch für große Volumina der Faulbehälter. Die Ausgestaltung der Tragsäule als Rohr oder als Formrohr ist besonders vorteilhaft, diese kann jedoch auch eine Gitterstruktur aufweisen.

Die Gestaltung des Faulbehälters, rund oder quadratisch mit einem steilen Böschungswinkel, ergibt ein günstiges Verhältnis Volumen/Oberfläche und eine gute Entleerung bei gleichzeitig einfacher Bauform.

Der ebenfalls kegelige oder pyramidenförmige Aufbau der Schutzabdeckung und des Foliengasbehälters wird durch Verwendung einer Tragsäule sehr vereinfacht, wobei die Einhaltung eines steilen Erhebungswinkels bei ebenfalls günstigem Volumen/(Oberflächenverhältnis möglich ist.

Die Verwencung des Pyramidenwinkels als Erhebungswinkel für Schutzabdeckung und Foliengasbehälter ergibt energetisch besonders günstige Verhältnisse für die Trocknungswirkung des Foliengasbehälters auf das abgezogene Biogas. Dabei erfolgt innerhalb der pyramidenförmigen Schutzabdeckung, innerhalb des Foliengasbehälters eine energetische Aufladung des Gases unter dem Einfluß der gebündelten kosmischen Strahlung, durch die die molekulare Struktur des Gases verdichtet wird und zu einer Vergrößerung des Brennwertes des Biogases führt.

Im pyramidenförmigen Faulraum des Faulbehälters, unterhalb der pyramidenförmigen Schutzabdeckung, wirkt das kosmische Strahlungsfeld positiv auf die mikrobiellen Umsetzungsprozesse in der auszufaulenden Flüssigkeit ein, durch die der Wasserstoffanteil im Biogas vergrößert und der Brennwert dadurch erhöht wird.

Durch Einblasen einer geringen Menge Luft in der Höhe des Flüssigkeitsspiegels, etwa 1% bezogen auf das Biogas, wird die Entschwefelung des Biogases durch Propagierung von Entschwefelungsbakterien bewirkt.

Für den störungsfreien Ablauf des Gärungsvorganges und dessen optimale Führung ist es wichtig, die Gärungstemperatur möglichst konstant und an die Bedürfnisse der Mikroorganismen angepaßt einzuhalten und dafür Sorge zu tragen, daß in den Randschichten die Temperatur nicht zu stark abfällt, wofür die Ausgestaltung der Behälterwand als Doppelwand mit Isolationszwischenlage besonders vorteilhaft ist. Weiters ist die Anbringung eines Heizregisters im unteren Bereich des Faulbehälters für die Durchführung eines gleichmäßigen Gärungsvorganges wichtig, wobei vorteilhafterweise die anfallende Abwärme aus dem Biogas Verwendung finden kann.

Für die stabile Anbringung notwendiger Behälterinstallationen und die Verankerung der zentralen Tragsäule ist die Herstellung eines Teilfundamentes, das an einer Stelle des Faulbehälters von dessen oberen Rand bis zum Boden geführt ist, ausreichend.

Bei der Einspeisung der auszufaulenden Flüssigkeit, wie Gülle oder dergl., ist die Einbringung in Bodennähe günstig um keinen Gaseintrag oder unkontrollierte Wirbelbildung zu erhalten. Der Abzug der ausgefaulten Flüssigkeit muß vom Boden her erfolgen, wobei die Führung des Austragsrohres über den oberen Rand die Verwendung einer selbstansaugenden Pumpe notwendig macht, während die Führung im Erdboden einen geringeren technischen Aufwand bedeutet.

Eine weitere Vereinfachung wird erhalten, wenn auch das Einspeisungsrohr im Erdboden geführt ist oder dieses und das Austragsrohr in einer einzigen Leitung vereinigt sind.

Die einwandfreie Befestigung der Folien des Faulbehälters und der Schutzabdeckung sowie des Gasbehälters, die zum Teil gasdicht sein muß, soll sorgfältig und einwandfrei ausgeführt sein, wofür einige Bauvarianten vorgesehen sind, die je nach der Größe des Faulbehälters und seines Aufbaus zur Anwendung gelangen.

Es ist die Anwendung eines Betonfundamentes als Randeinfassung sehr einfach und zuverlässig und gegen chemische Angriffe verhältnismäßig leicht und einfach schützbar und es erlaubt die einfache Befestigung aller Folien an nur einer Randlinie, wodurch die Konstruktion und die Wartung wesentlich vereinfacht werden.

Besonders bei kleineren Anlagen ist auch die Anwendung eines Stahlprofils, besonders Edelstahl zur Vermeidung von Korrosion, möglich, an dem dann die Folien nach Bereichen aus denen sie zusammengeführt werden, zusammengefaßt oder überhaupt einzeln befestigt werden, wodurch bei Wartungsarbeiten nur einzelne Folien oder Folienbereiche gelöst werden müssen.

Die Ausbildung eines Rührwerkes im Faulbehälter wird besonders durch dessen Größe bestimmt und es kann entweder nur ein Seitenrührwerk oder aber auch nur ein Mittelrührwerk oder beide vorgesehen sein.

Das Seitenrührwerk ist vorteilhafterweise mit Rollen an Schienen verfahrbar und mit einem Zugwerk in unterschiedliche Flüssigkeitstiefen des Faulbehälters verfahrbar, um die Rührwirkung für eine gute Durchmischung optimal einstellen zu können.

Das Mittelrührwerk kann ein oder mehrere Rührwerke umfassen, die höhenverstellbar angebracht sind und deren Rührwerkzeuge waagrecht oder leicht schräg geneigt gerichtet arbeiten. Durch entsprechende Anordnung der Rührwerke ist es dabei möglich zusätzlich eine Rotation derselben zu erzeugen, wodurch die Rührwirkung insgesamt verbessert wird.
Es ist auch möglich, ein Mittelrührwerk in ein, in die Tragsäule eingebautes, Zwischenstück einzusetzen, wobei der Motor im Zwischenstück angebracht ist und die Rührwerkzeuge, insbesonders waagrecht nach außen gerichtet, in axialer Richtung, vorzugsweise nach oben arbeitend, umlaufen.

Das Rührwerk kann auch mit einem rohrförmigen Rotor, an der Tragsäule umlaufend gelagert, ausgebildet sein.

Zur Zerstörung einer sich bildenden festen Decke auf der Gärflüssigkeit besteht auch die Möglichkeit ein Zuführungsrohr in der Tragsäule bis oberhalb der Flüssigkeitsoberfläche zu führen und dort durch eine oder mehrere Öffnungen austreten zu lassen, die auch unterschiedliche Ausrichtungen besitzen können um einen großen Teil der Oberfläche zu überstreichen.

Um die Wärmehaltung des Faulbehälters zu verbessern, ist die Anbringung einer thermischen Abdeckung im Abstand oberhalb der Flüssigkeitsfläche vorteilhaft durch die auch gleichzeitig die Feuchtigkeitsabfuhr von der Flüssigkeitsoberfläche vermindert wird, weil dadurch die Gaszirkulation in diesem Teil des Gasbehälters erheblich herabgesetzt wird.

Sehr vorteilhaft ist es dabei, die tragende Funktion und die Isolationsfunktion dieser Abdeckung zu trennen, wobei zumindest der wärmedämmende Teil aus vorgefertigten Bauelementen hergestellt sein kann, der dadurch sehr einfach verlegbar ist.

Diese thermische Abdeckung ruht außen auf der Behälterwand und ist innen an der Tragsäule befestigt.

Zum Schutz des wärmedämmenden Teiles der Abdeckung gegen Feuchtigkeit ist die Anbringung von Schutzfolien von Vorteil mit denen die einzelnen Bauelemente eingehüllt oder die gesamte Abdeckung umhüllt ist, die dann in diesem Fall vorteilhafterweise an der Randeinfassung des Faulbehälters befestigt wird.

Die Befestigung der thermischen Abdeckung an der Tragsäule kann in vorteilhafterweise an einem Verbindungsflansch geschehen, der den unteren und den oberen Teil der Tragsäule trennt.

Das entwickelte Biogas läßt man entweder durch Öffnungen an der Fahne des Verbindungsflansches oder zwischen den Bauelementen der thermischen Abdeckung hindurchtreten, wodurch der Bauaufwand gering gehalten wird.

Die Befestigung der Folien der Behälterwand des Gasbehälters und der Schutzabdeckung wird vorzugsweise mit einer einzigen Klemmverbindung an der Oberseite oder an der Außenseite der Randeinfassung des Faulbehälters hergestellt, wobei eine Klemmplatte die Folie in ihrer Lage mit einer Verschraubung an der Randeinfassung geklemmt hält.

Für den Abfluß von Kondenswasser aus dem Zwischenraum zwischen Foliengasbehälter und Schutzabdeckung können durch Einlagen, z.B. Kunststoffgitterstreifen oder dergl., Öffnungen geringen Querschnittes hergestellt sein.
Für den Durchtritt des Biogases nach oben in den Foliengasbehälter durch die thermische Abdeckung hindurch können solche Öffnungen in ähnlicher Weise hergestellt werden.

Besonders schonend wird die Klemmverbindung gestaltet, wenn eine weiche, widerstandsfähige Einlage, z.B. aus Schaumgummi oder Kunststoffschaum oder dergl., eingelegt ist, um die die Folie herumgeführt wird.

Die Anbringung von mehreren aufgeteilten Klemmverbindungen zur Befestigung der Folien ist bei Metallprofilen günstiger, weil dadurch eine besonders zuverlässige Befestigung hergestellt werden kann.

Durch die Anwendung einer Kantenschiene, insbesonders aus Metall, kann bei Verwendung von Betonprofilen die Randeinfassung für die Klemmverbindung der Folien besonders schonend gestaltet werden.

Durch das Einfärben der Abdeckfolie für die Schutzabdeckung und ihre Verstärkung mit Gewebeeinlagen wird ihre Haltbarkeit wesentlich verlängert.

Für die kompakte Gestaltung der Anlage und ihre Wartung ist die Anbringung einer Arbeitsplattform oberhalb des Foliengasbehälters im Bereich der Spitze der Schutzabdeckung von Vorteile weil dort eine Maschine für die Wärmekraftkopplung oder ein Heizgerät aufgestellt werden kann die das Biogas direkt verarbeitet und weil die Anbringung eines Wärmespeichers, z.B. für Warmwasser, oberhalb der Arbeitsplattform, in der Spitze der pyramidenförmig oder kegelig geformten oberen Schutzabdeckung, kurze Leitungen ergibt und statisch vorteilhaft zu gestalten ist. Die Abführung dieser gespeicherten Wärme wird vorteilhafterweise entweder an der Tragsäule entlang nach unten und durch ein Mannloch nach außen oder außen an der Schutzabdeckung entlang vorgenommen. Es ist aber auch möglich das erzeugte Biogas direkt nach außen abzuführen und zu verwerten.
Zum Ausgleich der Volumsänderungen des Foliengasbehälters ist es von Vorteil, wenn am oberen Ende der Schutzabdeckung, z.B. an der Arbeitsplattform, eine Ausgleichsöffnung im bereich zwischen der Abdeckfolie der Schutzabdeckung und der Gaspehälterfolie angebracht ist und zur Sicherung letzterer eine Ausblasleitung mit Abfackelung, mit einem Überdruckventil von etwa 1 bis 2 mbar vorgesehen ist.

Das im Foliengasbehälter gespeicherte und insbesonders angereichte und getrocknete, Biogas tritt durch Öffnungen in der Tragsäule, oberhalb des Verbindungsflansches in eine in dieser angebrachten Waschsäule über, die mit Material zur Abtrennung des Wassers und gegebenenfalls zur Abtrennung von umgesetzten Schwefelverbindungen gefüllt ist, und gelangt vom oberen Ende der Tragsäule unmittelbar in die Leitung zur Wärmekraftkopplungsmaschine oder zu einem dort aufgestellten Heizkessel oder in eine Leitung zur Abfuhr des Biogases nach außen. Es wird dadurch eine sehr einfache, platzsparende Behandlung des Biogases ermöglicht.

Die Gasbehälterfolie ist vorteilhafterweise an der Tragsäule mit einer Klemmverbindung gasdicht befestigt, während die Folie der Schutzabdeckung außen an der Arbeitsplattform befestigt ist.

Der obere Teil der Schutzabdeckung bis zur Spitze desselben besteht aus einem festen Material, beispielsweise aus Blech, weil die auf der Arbeitsplattform unter der Schutzabdeckung befindlichen Installationen einfacher angebracht und gewartet werden können.

Es kann aber auch die gesamte Schutzabdeckung aus einer festen Verkleidung, wie Mineralplatten oder Blech oder dergl., bestehen.

Die Folie der Schutzabdeckung wird nach unten zu von mehreren Abspannseilen gehalten, die in einfacher und verläßlicher Bauweise von im Boden angebrachten Verankeruhgen, z.B. in Form von Bohrpfählen, abgespannt sind.

Für den Zugang unter die Schutzabdeckung und zum Foliengasbehälter sowie zum Faulbehälter ist vorteilhafterweise im Bereich des Betonfundamentes ein gasdicht abschließbares Mannloch sowohl in der Schutzabdeckung als auch im Foliengasbehälter vorgesehen.

Ein Faulbehälter mit der beschriebenen Schutzabdeckung und dem Foliengasbehälter ist geeignet auch andere biogene Abfälle als Gülle, z.B. auch zerkleinerte feste organische Abfälle, zu verarbeiten.

Zur kontinuierlichen Verarbeitung von biogenen Abfällen in Flüssigkeit ist die Anwendung von zwei Faulbehältern der beschriebenen Art von Vorteil, die jeweils abwechselnd befüllt oder ausgefault und entleert werden, wobei die Nachbehandlung in einem größeren, wenigstens doppelt so großen Faulbehälter der beschriebenen Art geschehen kann, bei der die gewöhnlich noch verbleibenden etwa 10% des unzersetzten organischen Materials abgebaut wird, bevor eine Entleerung erfolgt.

Die Erfindung wird an Hand der Zeichnung eines Ausführungsbeispieles beschrieben.
Es zeigt:

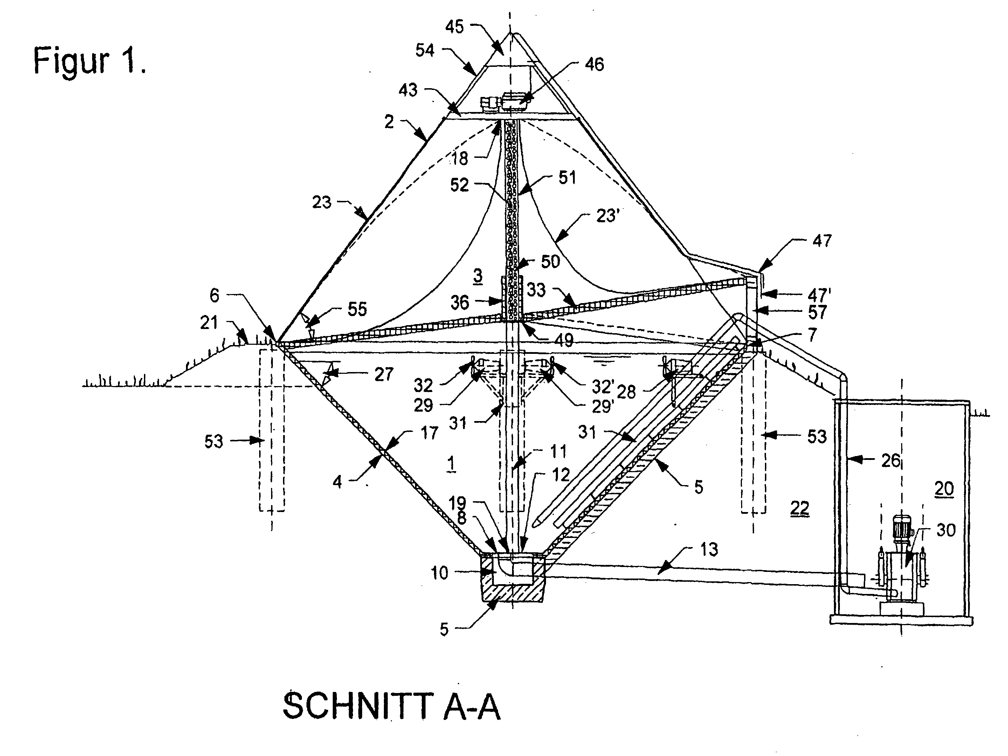

- Fig. 1: einen Biogasfermenter in Seitenansicht, geschnitten;

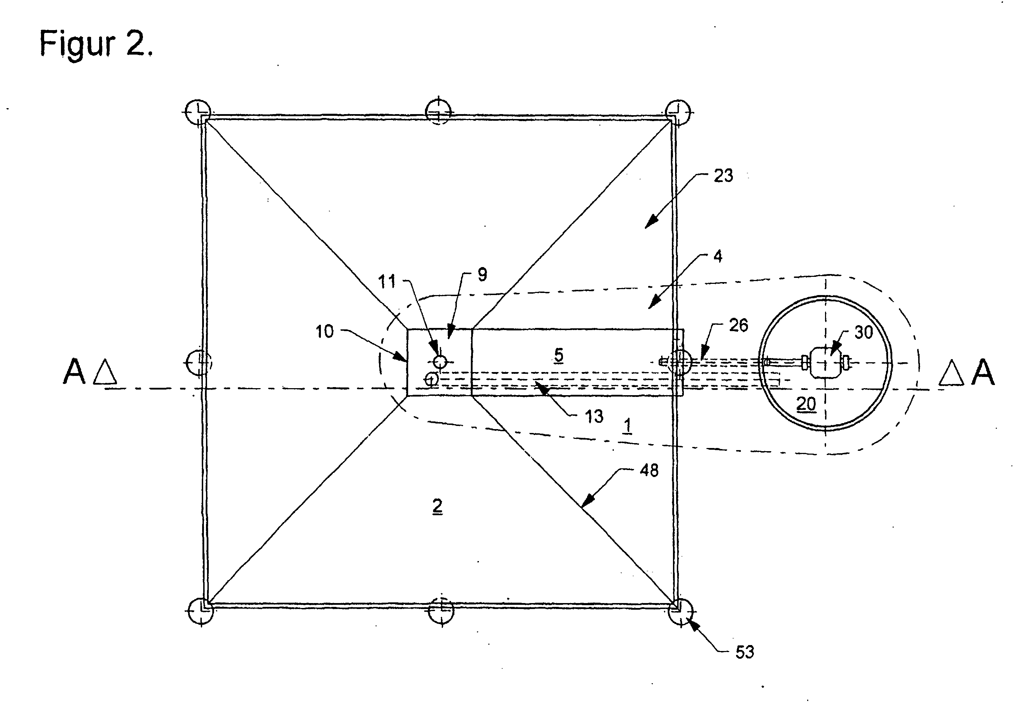

- Fig. 2: eine Draufsicht auf den Biogasfermenter nach Fig. 1, mit teilweise aufgerissener Abdeckung;

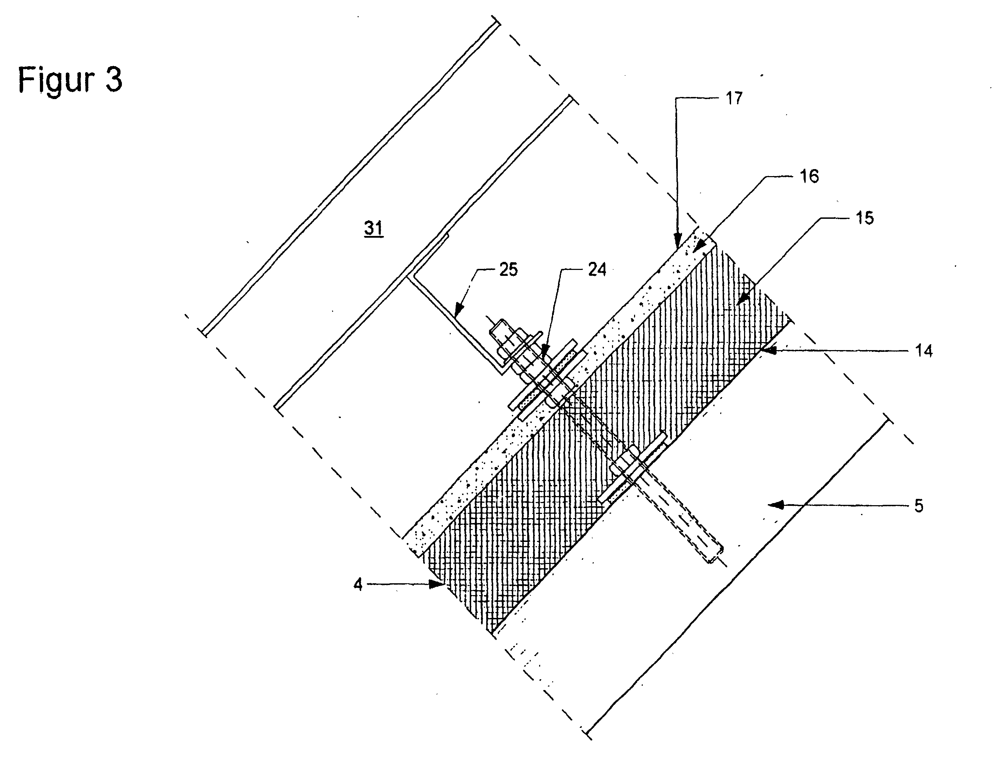

- Fig. 3: einen Ausschnitt aus der Behälterwand des Faulbehälters;

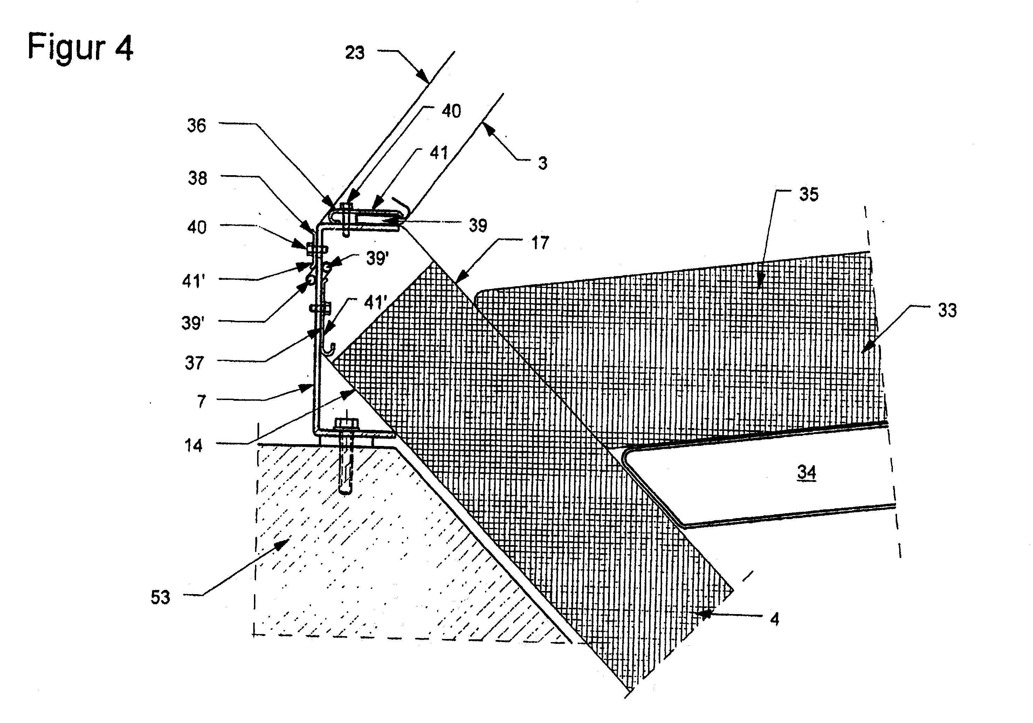

- Fig. 4: den oberen Rand des Faulbehälters mit einer Randeinfassung aus Metallprofil mit Klemmverbindungen für Folien, im Schnitt;

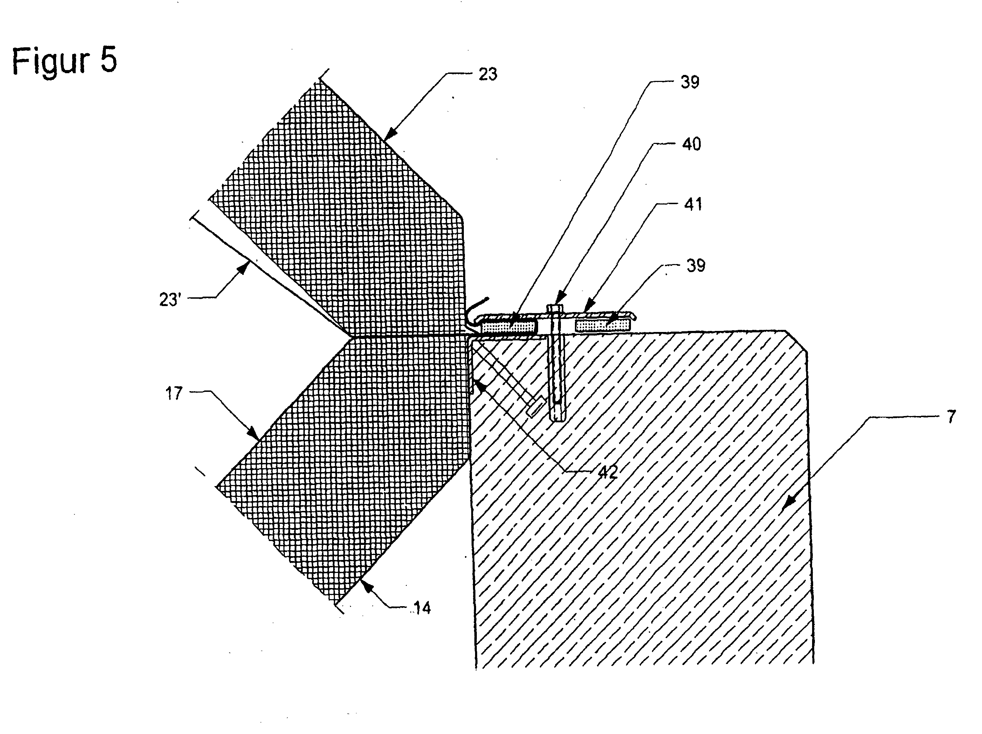

- Fig. 5: den oberen Rand des Faulbehälters mit einer Randeinfassung aus Betonprofil, mit innen liegender Klemmverbindung für die Folien, im Schnitt;

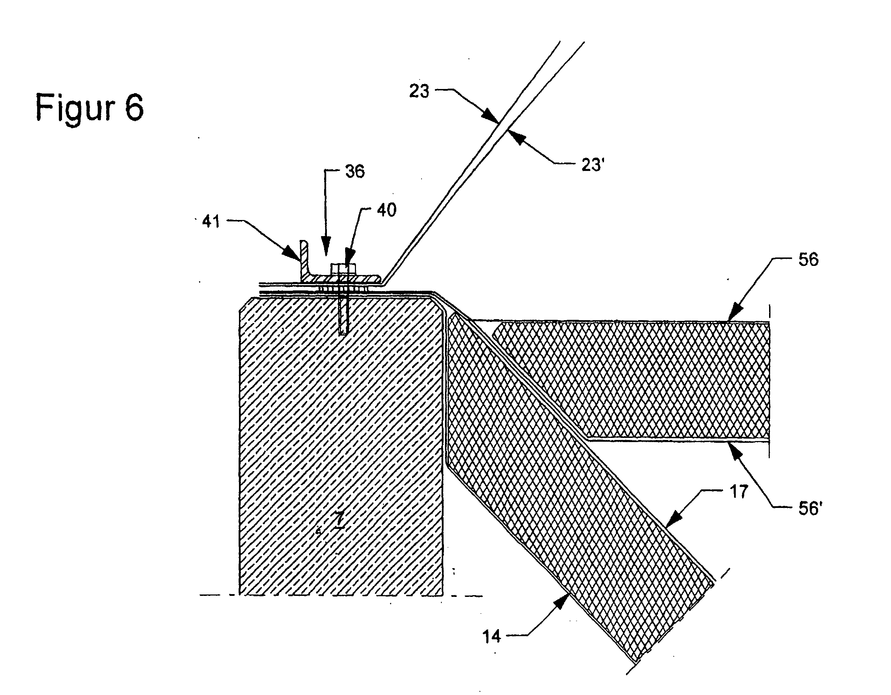

- Fig. 6: den oberen Rand des Faulbehälters mit einer Randeinfassung aus Betonprofil, mit einer an der Oberseite angeordneten Klemmverbindung für die Folien, im Schnitt.

Eine Biofermenteranlage besitzt einen Faulbehälter 1 der von einer Schutzabdeckung 2 überdeckt ist, unter der ein Foliengasbehälter 3 den Faulbehälter 1 überspannt.

Der Faulbehälter 1 wird von einer Behälterwand 4 gebildet, die auf einem, in einer Bodeneintiefung ausgebreitetem, Bodenvlies verlegt ist, wobei der Faulbehälter 1 einen sich nach unten zu verjüngenden quadratisch-pyramidenförmigen oder kegeligen Aufbau besitzt, mit einem Neigungswinkel 27 von 45°.

An einer Stelle des Faulbehälters 1 ist ein Betonfundament 5 hergestellt, das vom oberen Rand 6 des Faulbehälters 1 bis zu dessen Boden 8 verläuft, wo ein Austragschacht 10 gebildet ist, der mit einer ebenen Bodenplatte 9 abschließt.

In dem Austragschacht 10 endet ein Austragsrohr 13, das durch den Erdboden 22 in eine als Schacht ausgeführte Pumpenkammer 20 und an eine Pumpe 30 einer Pumpanlage angeschlossen ist.

Auf der Bodenplatte 9 ist eine Tragsäule 11 für die Schutzabdeckung 2 und den Foliengasbehälter 3 mit einer an ihrem unteren Ende 19 befestigten Verankerungsplatte 12 verankert.

Aus der Pumpenkammer 20 führt ein Einspeisungsrohr 26 für die zu verarbeitende Gülle von der Pumpe 30 über den oberen Rand 6 in den Faulbehälter 1 und im Abstand vom Betonfundament 5 an Profilträgern 25 gehalten an der Behälterwand 4 nach unten, bis nahe an den Boden 8 desselben.
Das Einspeisungsrohr 26 ist dabei im Betonfundament 5 mit durch die Behälterwand 4 abgedichtet hindurchgeführten Verankerungsverschraubungen befestigt.

Die Behälterwand 4 besteht aus einer Bodenfolie 14 auf der eine Isolationsschicht 15 aus einem gebräuchlichen Isola-tionsmaterial aufgebracht ist und die im unteren Teil der Behälterwand 4 mit einem aufgelegten Heizregister 16 überdeckt ist, auf der eine Behälterfolie 17 aufgelegt die Behälterwand zum Inneren des Faulbehälters 1 hin abdichtet.

Im Faulbehälter 1 ist ein Seitenrührwerk 28 angeordnet, das mit Rollen auf einer Rührwerksführung 31 aus Schienen verfahrbar ist, die an durch die Behälterwand 4 abgedichtet hindurchgeführten Verankerungsverschraubungen 24, die Profilträger 25 tragen, befestigt ist, wobei das Seitenrührwerk 28 mit einem nicht dargestellten Zugwerk, wie einer Seilwinde, der Höhe nach im Faulbehälter 1 verfahrbar ist und mit dem Rührwerkzeug 32 in waagrechter Richtung arbeitend angeordnet ist.

An der Tragsäule 11 ist ein Mittelrührwerk 29,29' aus zwei Rühreinheiten an einer Rührwerksführung 31 höhenverstellbar gelagert, wobei die Rührwerkzeuge 32,32' ebenfalls waagrecht arbeitend angeordnet sind.

Am oberen Rand 6 des Faulbehälters 1 ist im Abstand oberhalb der Flüssigkeit eine thermische Abdeckung 33 angeordnet, die aus einem tragenden Teil 34 auf dem ein wärmedämmender Teil 35 aus vorgefertigten Bauelementen aufgeiegt ist, besteht.

Der wärmedämmende Teil 35 wird von einer unteren Schutzfolie 56 und einer oberen Schutzfolie 56' abgedeckt. die an der Tragsäule 11 und an der Randeinfassung 7 befestigt sind. Der wärmedämmende Teil 35 ist an der Tragsäule 11 mit einem rohrförmigen Stück nach oben gezogen.
Die thermische Abdeckung 33 liegt innen auf dem Verbindungsflansch 49 auf und ruht außen auf der Behälterwand 4.

Oberhalb des Verbindungsflansches 49 sind im oberen Teil 51 der Tragsäule 11 Öffnungen angebracht, durch die das Biogas aus dem Foliengasbehälter 3 in eine, in der Tragsäule 11 befindliche, Waschsäule 50 eintreten kann und in deren Waschfüllung 52 von Wasser und anderen unerwünschten Inhaltsstoffen befreit wird, worauf es am oberen Ende 18 der Tragsäule 11 durch eine Zuleitung in die thermoelektrische Wärmekraftanlage 46 eintritt.

Der obere Rand 6 des Faulbehälters 1 befindet sich etwa an der Bodenoberfläche 21 und besitzt eine Randeinfassung 7, an der die Bodenfolie 14 und die Behälterfolie 17 der Behälterwand 4, sowie die Gasbehälterfolie 23' des Foliengasbehälters 3 und die Abdeckfolie 23 der Schutzabdeckung 2 befestigt sind.

Die Tragsäule 11 trägt an ihrem oberen 18 eine Arbeitsplattform 43 an deren Außenrand die schwarz eingefärbte Abdeckfolie 23 befestigt ist während innen an der Tragsäule 11 die Gasbehälterfolie 23' gasdicht angebracht ist.
Die Arbeitsplattform 43 ist nach oben zu bis zur Spitze 44 der pyramidenförmigen Schutzabdeckung 2 mit derem oberen, mit einer festen Verkleidung versehenen, Teil 54 abgedeckt.

An der Spitze 44 ist ein Wärmespeicher 45 für die Wärme aus der thermoelektrischen Wärmekraftanlage 46 angebracht, der mit Zu- und Ableitungen 47,47' für das Wärmeübertragungsmittel verbunden ist, die außen an der Schutzabdeckung 2 herabgeführt sind.

Die Schutzabdeckung 2 ist mit ihrer Abspannung 48 an mehreren Verankerungen 53, die als Bohrpfähle ausgeführt sind, gehalten und besitzt im Bereich des Betonfundamentes 5 ein Mannloch 57, an das auch der Foliengasbehälter 3 gasdicht angeschlossen ist.

Die Folien 14 und 17 der Behälterwand 4 sowie die Folien 23 und 23' der Schutzabdeckung 2 und des Foliengasbehälters 3 und die Folien 56 und 56' der thermischen Abdeckung 33 sind mit einer Klemmverschraubung 36 an der Oberseite eines Betonprofils der Randeinfassung 7 des Faulbehälters 1 befestigt, in dem sie von einer Klemmplatte 41 vermittels einer Verschraubung 40, die in der Randeinfassung 7 verschraubt ist, eingeklemmt sind.

Bei einer anderen Ausführungsart ist die Bodenfolie 14 der Behälterwand 4 an der Innenseite eines Stahlprofils der Randeinfassung 7 mit einer Klemmverschraubung 37, bestehend aus einer Klemmplatte 41 und einer Verschraubung 40, niedergehalten befestigt, nachdem sie vorher um eine nachgiebige Einlage 39', z.B. einem Plastikschlauch, herumgeführt ist. In gleicher Weise ist die Abdeckfolie 23 der Schutzabdeckung 2 an der Außenseite des Stahlprofils der Randeinfassung 7 mit einer Klemmverschraubung 38 befestigt.

Die Behälterfolie 17 der Behälterwand 4 ist zusammen mit der Gasbehälterfolie 23' um eine flache, nachgiebige Einlage 39 herumgeführt und mit einer Klemmplatte 41 einer Klemmverschraubung 36 dichtend niedergehalten, die mit Verschraubungen 40 befestigt ist.

Für die Abfuhr von Kondenswasser aus dem Bereich zwischen dei Abdeckfolie 23 und der Gasbehälterfolie 23' sowie aus dem Zwischenraum zwischen dieser und der thermischen Abdeckung 33 sind Öffnungen geringen Querschnittes, z.B. durch Zwischeneinlagen aus Kunststoffgittern oder ähnlichem, hergestellt.

Bei einer abgewandelten Folienbefestigung an einer Randeinfassung 7 aus Betonprofil ist die Klemmverschraubung 36 in der beschriebenen Art an der Oberseite der Randeinfassung 7 angeordnet, die gegen eine im Betonprofil verankerte Kantenschiene 42 an der Innenseite, zum Faulbehälter 1 hin, abgestützt ist.

### Bezugszeichenliste

- 1: Faulbehälter
- 2: Schutzabdeckung
- 3: Foliengasbehälter
- 4: Behälterwand des Faulbehälters 1
- 5: Betonfundament am Faulbehälter 1
- 6: oberer Rand des Faulbehälters 1
- 7: Randeinfassung des Faulbehälters 1
- 8: Boden des Faulbehälters 1
- 9: Bodenplatte des Faulbehälters 1
- 10: Austragsschacht des Faulbehälters 1
- 11: Tragsäule für die Schutzabdeckung 2 und den Foliengasbehälter 3
- 12: Verankerungsplatte der Tragsäule 11
- 13: Austragsrohr des Faulbehälters 1
- 14: Bodenfolie der Behälterwand 4 des Faulbehälters 1
- 15: Isolationsschicht der Behälterwand 4
- 16: Heizregister der Behälterwand 4
- 17: Behälterfolie der Behälterwand 4
- 18: oberes Ende der Tragsäule 11
- 19: unteres Ende der Tragsäule 11
- 20: Pumpenkammer
- 21: Bodenoberfläche
- 22: Erdboden
- 23: Abdeckfolie der Schutzabdeckung 2
- 23': Gasbehälterfolie
- 24: Verankerungsverschraubung für das Einspeisungsrohr 26
- 25: Profilträger
- 26: Einspeisungsrohr für die Gülle
- 27: Neigungswinkel der Behälterwand 4
- 28: Seitenrührwerk
- 29,29': Mittelrührwerk
- 30: Pumpe der Pumpanlage
- 31: Rührwerksführung
- 32,32': Rührwerkszeuge
- 33: thermische Abdeckung des Faulbehälters 1
- 34: tragender Teil der thermischen Abdeckung 33
- 35: wärmedämmender Teil der thermischen Abdeckung 33
- 36: Klemmverschraubung für die Behälterfolie 17 und die Folie des Foliengasbehälters 3
- 37: Klemmverschraubung für die Bodenfolie 14 der Behälterwand 4
- 38: Klemmverschraubung für die Abdeckfolie 23 der Schutzabdeckung 2
- 39,39': nachgiebige Einlage der Klemmverschraubung 36,37,38
- 40: Verschraubung der Klemmverschraubung 36,37,38
- 41,41': Klemmplatte der Klemmverschraubung, 36,37,38
- 42: Kantenschiene der Randeinfassung 7
- 43: Arbeitsplattform der Tragsäule 11
- 44: Spitze der Schutzabdeckung 2
- 45: Wärmespeicher
- 46: thermoelektrische Wärmekraftanlage
- 47,47': Zu- und Ableitung für das Wärmeübertragungsmittel
- 48: Abspannung der Schutzabdeckung 22
- 49: Verbindungsflansch der Tragsäule 11
- 50: Waschsäule in der Tragsäule 11
- 51: oberer Teil der Tragsäule 11
- 52: Waschfüllung der Waschsäule 50
- 53: Verankerung der Abspannung 48
- 54: oberer Teil der Schutzabdeckung 2
- 55: Erhebungswinkel der Schutzabdeckung 2
- 56: obere Schutzfolie des wärmedämmenden Teils 35 der thermischen Abdeckung 33
- 56': untere Schutzfolie des wärmedämmenden Teils 35 der thermischen Abdeckung 33
- 57: Mannloch in der Schutzabdeckung 2 und in dem Foliengasbehälter 3

## Patentansprüche

1. Biofermenteranlage mit wenigstens einem Faulbehälter für eine auszufaulende Flüssigkeit, insbesondere Gülle, mit einer dachförmigen, abgestützten Schutzabdeckung, innerhalb der ein gasdichter Foliengasbehälter den Faulbehälter, zu diesem hin offen, überdeckt und mit einem Einspeisungs- und einem Austragsrchr und einer Pumpanlage für die Gülle, mit einer Heizungseinrichtung und wenigstens einem Rührwerk im Faulbehälter ausgestattet und mit wenigstens einer Abführungsleitung für einen Energieträger versehen ist, **dadurch gekennzeichnet, daß** eine, insbesonders als Rohr oder Profilrohr ausgebildete Tragsäule (11) mittig im Faulbehälter (1) angeordnet ist, an deren oberen Ende (18) die äußere Schutzabdeckung (2) befestigt und das obere Ende des Foliengasbehälters(3) gasdicht angebracht ist und die mit dem unteren Ende (19) im Faulbehälter (1), insbesonders auf dem Boden (8) desselben, verankert ist, daß der Faulbehälter (1) kegelig oder quadratisch pyramidenförmig, vom oberen Rand (6) nach unten zu, insbesonders von der Höhe der Bodenoberfläche (21) ausgehend, sich verjüngend geformt ist und seine Behälterwand (4) im wesentlichen von einer Bodenfolie (14), die auf einem Bodenvlies, vorzugsweise unmittelbar, auf dem Erdboden (22) aufgelegt ist und einer die Innenwand bildenden Behälterfolie (17) gebildet ist, wobei der Neigungswinkel (27) der Behälterwand (4) zur Waagrechten zwischen 30° und 50° und vorzugsweise etwa 35° bis 45° beträgt und daß die Schutzabdeckung (2) des Faulbehälters (1) kegelig oder quadratisch pyramidenförmig, nach oben zu, insbesonders von der Höhe des Erdbodens, vom oberen Rand (6) des Faulbehälters (1) ausgehend, sich verjüngend geformt ist, wobei der Erhebungswinkel (55) zwischen 40° und 60°, vorzugsweise mehr als 45° beträgt.

2. Biofermenteranlage nach Anspruch 1, **dadurch gekennzeichnet, daß** die Schutzabdeckung (2) des Faulbehälters (1) einen Erhebungswinkel (55) von zwischen 50° und 55° und vorzugsweise von etwa 51° bis 52° aufweist.

3. Biofermenteranlage nach Anspruch 1 oder 2, **dadurch gekennzeichnet, daß** die Schutzabdeckung (2) des Faulbehälters (1) einen Erhebungswinkel (55) von im wesentlichen 51,87°, dem Neigungswinkel der "Großen Pyramide" entsprechend aufweist und einen nach unten sich verjüngenden, quadratisch pyramidenförmigen Faulbehälter (1) mit einem Neigungswinkel (27) von mehr als 30° und vorzugsweise wenigstens 45° überdeckt.

4. Biofermenteranlage nach Anspruch 1 oder 3, **dadurch gekenneichnet, daß** die Behälterwand (4) des Faulbehälters (1) von einer Bodenfolie (14) gebildet ist, auf der eine Isolationsschicht (15), insbesonders aus PU-Schaum, durchgehend aufgebracht ist, auf der, wenigstens über einen Teil der Behälterwand (4), vorzugsweise einem unteren Teil derselben, Heizungsregister (16) einer Heizungseinrichtung, insbesonders als Warmwasser-Rohrregister, aufgelegt sind und als Innenwand eine aufliegende Behälterfolie (17) dichtend aufgebracht ist.

5. Biofermenteranlage nach Anspruch 1, 3 oder 4, **dadurch gekennzeichnet, daß** wenigstens an einer Stelle des Faulbehälters (1), bei einem quadratischen Umriß des Faulbehälters (1), vorzugsweise in der Mitte einer Seite desselben, ein vom oberen Rand (6) des Faulbehälters (1) nach unten bis zu dessen Boden (8) verlaufend ein Betonfundament (5) oder dergl. eingebaut ist und mit einer ebenen Bodenplatte (9), insbesonders in einem vertieften Abzugsschacht (10) angeordnet, abschließt, auf der die Tragsäule (11) mit einer Verankerungsplatte (12) oder dergl. befestigt ist.

6. Biofermenteranlage nach Anspruch 1, oder 5, **dadurch gekennzeichnet, daß** das Einspeisungsrohr (26) vom oberen Rand (6) des Faulbehälters (1) her, im wesentlichen bis an den Boden (8) oder zumindest in den Bodenbereich desselben reicht und, vorzugsweise im Abstand (5) von der Behälterwand (4), insbesonders auf in das Bodenfundament (5) eingesetzten, durch die Behälterwand (4) abgedichtet hindurchgeführte Ankerverschraubungen (24) über Befestigungsprofile (25) befestigt ist und mit einer Pumpe (39) einer Pumpanlage in der, vorzugsweise als Pumpenschacht ausgebildeten Pumpenkammer (20) verbunden ist und das Austragsrohr (13) in der ebenen Bodenplatte (9), insbesonders in einem vertieften Abzugsschacht (10) angeordnet, mündet und vorzugsweise an der Behälterwand (4) entlang nach oben über den oberen Rand (6) des Faulbehälters (1) oder unterhalb desselben im Erdboden in eine Pumpenkammer (20) geleitet ist.

7. Biofermenteranlage nach Anspruch 1, 5 oder 6 **dadurch gekennzeichnet, daß** das Austragsrohr (13) gleichzeitig als Einspeisungsrohr vorgesehen ist.

8. Biofermenteranlage nach Anspruch 1 bis 7, **dadurch gekennzeichnet, daß** der obere Rand (6) des Faulbehälters (1) mit einer Randeinfassung (7) aus einem Betonprofil, versehen ist, an der die Bodenfolie (14) und die Behälterfolie (17) der Behälterwand (4) und die Abdeckfolie (23) der Schutzabdeckung (2) und die Gasbehälterfolie (23') des Foliengasbehälters (3), vorzugsweise von außen her, dichtend befestigt sind.

9. Biofermenteranlage nach Anspruch 1 bis 7, **dadurch gekennzeichnet, daß** der obere Rand (6) des Faulbehälters (1) mit einer Randeinfassung (7) aus einem Profil, insbesonders aus Edelstahl oder dergl., versehen ist, das vorzugsweise aus einem nach innen offenen U-Profil besteht.

10. Biofermenteranlage nach einem der Ansprüche 1 bis 9, **dadurch gekennzeichnet, daß** wenigstens ein Seitenrührwerk (28) an der Behälterwand (4), insbesonders auf dem Betonfundament (5), von oben, unmittelbar unterhalb des oberen Randes (6) des Faulbehälters (1), bis nach unten, nahezu an dessen Boden (8), vorzugsweise mit Rollen auf einer Rührwerksführung (31), in Form von Bahnen, verfahrbar ist, die an durch die Behälterwand (4) abgedichtet hindurchgeführte Ankerverschraubunzen (24) über Befestigungsprofile (25) befestigt ist und motorisch angetrieben von einem Zugwerk, vorzugsweise von einer am oberen Rand (6) des Faulbehälters (1) insbesonders außerhalb der Schutzabdeckung (2), aufgestellten Seilwinde bewegbar ist.

11. Biofermenteranlage nach einem der Ansprüche 1 bis 10, **dadurch gekennzeichnet, daß** an der Tragsäule (11) wenigstens ein Mittelrührwerk (29) angeordnet und an einer Rührwerksführung (31), insbesonders mit im wesentlichen radial zur Tragsäule und waagrecht arbeitend ausgerichteten Rührwerkswerkzeugen (32), angeordnet sind und daß bei zwei Mittelrührwerken (29,29') diese in der Ausrichtung der Rührwerkswerkzeuge (32,32') zur Achse der Tragsäule (11) beiderseits versetzt angeordnet sind oder daß die Ausrichtung der Rührwerkswerkzeuge teilweise oder vollständig tangential zur Achse der Tragsäule (11) vorgesehen ist.

12. Biofermenteranlage nach einem der Ansprüche 1 bis 11, **dadurch gekennzeichnet, daß** in der Tragsäule (11), insbesondere zwischen Flanschen, ein zentrales Mittelrührwerk eingebaut ist, das insbesonders rohrförmig ausgebildet ist und dessen Rührwerkzeuge vorzugsweise im wesentlichen waagrecht ausgerichtet, in axialer Richtung insbesonders nach oben gerichtet arbeitend angeordnet sind, deren Antriebsmotor vorzugsweise axial zwischen die Flansche der Tragsäule (11) eingesetzt ist.

13. Biofermenteranlage nach einem der Ansprüche 1 bis 12, **dadurch gekennzeichnet, daß** die Tragsäule (11) direkt oder über ein in dieser geführtes Zuführungsrohr, das mit einer Umwälzpumpe der Pumpanlage verbunden ist, für die Umwälzung der Gülle vorgesehen ist, die im Bereich des oberen Randes (6), insbesonders in einem Abstand oberhalb des höchsten Standes der Flüssigkeitsoberfläche im Faulbehälter (1) durch wenigstens eine Öffnung, vorzugweise im wesentlichen waagrecht oder flach nach unten gerichtet, ausbringbar ist.

14. Biofermenteranlace nach Anspruch 13, **dadurch gekennzeichnet, daß** mehrere Öffnungen, vorzugsweise mit Düsen, insbesonders unterschiedlicher Strahlweite und mit einer Räumvorrichtung vor dem Einlauf der Öffnungen ausgestattet, vorgesehen sind.

15. Biofermenteranlage nach einem der Ansprüche 1 bis 14, **dadurch gekennzeichnet, daß** der Faulbehälter (1) am oberen Rand (6) mit einer thermischen Abdeckung (33) versehen ist, die an diesem und an der Tragsäule (11) befestigt ist und vorzugsweise im Abstand oberhalb des höchsten Standes der Gülleoberfläche im Faulbehälter (1) angebracht ist und insbesonders aus einem tragenden Teil (34) und einem darauf aufgelegten wärmedämmenden Teil (35) besteht und vorzugsweise der wärmedämmende Teil (35) aus vorgefertigten Bauelementen aufgebaut ist, die insbesonders mit Nut und Feder zusammengefügt sind und daß an der Tragsäule zusätzlich ein wärmedämmendes Rohrelement (36) auf die thermische Abdeckung (33) aufgesetzt ist.

16. Biofermenteranlage nach Anspruch 15, **dadurch gekennzeichnet, daß** jedes vorgefertigte Bauelement des wärmedämmenden Teiles (35) der thermischen Abdeckung (33) mit einer Schutzfolie (56,56') umhüllt ist oder daß mehrere oder alle vorgefertigten Bauelemente mit einer oberen Schutzfolie (56) und mit einer unteren Schutzfolie (56') abgedeckt sind, die beide an der Randeinfassung (7) und an der Tragsäule (11) befestigt sind.

17. Biofermenteranlage nach einem der Ansprüche 1 bis 16, **dadurch gekennzeichnet, daß** die thermische Abdeckung (33) mit dem tragenden Teil (34) und dem darauf verlegten oder verbundenen wärmedämmendem Teil (35) am oberen Rand (6) des Faulbehälters (1) auf der Behälterwand (4) aufliegend angeordnet ist.

18. Biofermenteranlage nach einem der Ansprüche 1 bi 17, **dadurch gekennzeichnet, daß** zumindest die Bodenfolie (14) und die Behälterfolie (17) der Behälterwand (4) die Gasbehälterfolie (23') des Foliengasbehälters (3) und die Abdeckfolie (23) der Schutzabdeckung (2) mit einer einzigen gasdichten Klemmverschraubung (36) zusammengefaßt befestigt sind, wobei die Klemmverschraubung (36) aus einer Verschraubung (40), die vorzugsweise an der Oberseite oder der Außenseite des Profils, insbesonders des Betonprofils, der Randeinfassung (7) eingeschraubt ist und aus einer Klemmplatte (41) besteht.

19. Biofermenteranlage nach Anspruch 1 oder 18, **dadurch gekennzeichnet, daß** zwischen der Abdeckfolie (23) und der. Gasbehälterfolie (23'), insbesonders nach außen führende, Durchtrittsöffnungen geringen Querschnittes, vorzugsweise durch eine Zwischenlage hergestellt, angebracht sind, derart daß Kondenswasser austreten kann.

20. Biofermenteranlage nach Anspruch 1 oder 18, **dadurch gekennzeichnet, daß** zwischen den vorgefertigten Bauelementen des wärmedämmenden Teiles (35) der thermischen Abdeckung (33) oder deren einhüllenden Schutzfolien und vorzugsweise am Verbindungsflansch (49), Durchtrittsöffnungen zum Foliengasbehälter (3) hin angebracht sind.

21. Biofermenteranlage nach einem der Ansprüche 1 bis 20, **dadurch gekennzeichnet, daß** die Bodenfolie (14) und die Behälterfolie (17) der Behälterwand (4) die Gasbehälterfolie (23' ) des Foliengasbehälters (3) und die Abdeckfolie (23) der Schutzabdeckung (2) mit mehreren gasdichten Klemmverschraubungen (36, 37, 38) an der Randeinfassung (7) des Faulbehälters (1) festgelegt sind.

22. Biofermenteranlage nach Anspruch 1 bis 21, **dadurch gekennzeichnet, daß** die Behälterfolie (17) und die Gasbehälterfolie (23') des Foliengasbehälters (3) gemeinsam um eine nachgiebige Einlage (39), insbesonders einer Platte aus Schaumgummi oder dergl., herumgeführt und vorzugsweise außen auf dem oberen Schenkel eines U-Profils der Randeinfassung (7) mit einer von einer Verschraubung (40) niedergehaltenen Klemmplatte (41) der Klemmverschraubung (36) gemeinsam befestigt sind.

23. Biofermenteranlage nach einem der Ansprüche 1 bis 22, **dadurch gekennzeichnet, daß** die Abdeckfolie (23) außen und die Bodenfolie (14) innen am U-Profil der Randeinfassung (7) jeweils um eine nachgiebige Einlage (39'), vorzugsweise aus einem Plastikschlauch oder dergl. bestehend, herumgeführt und mit einer von einer Verschraubung (40) niedergehaltenen Klemmplatte (41'), jeweils einzeln, befestigt sind.

24. Biofermenteranlage nach einem der Ansprüche 1 bis 23, **dadurch gekennzeichnet, daß** bei Ausbildung der Randeinfassung (7) als Betonprofil die Verschraubung (40) der Klemmverschraubung (36) mit einer Verankerung im Beton gehalten ist und die Kante des Betonprofils der Randeinfassung (7) mit einer im Beton verankerten Kantenschiene (42) als Dichtungsauflage versehen ist.

25. Biofermenteranlage nach einem der Ansprüche 1 bis 24, **dadurch gekennzeichnet, daß** die Folien, insbesonders die Abdeckfolie (23) und die Folie des Foliengeasbehälters (3) dunkel, lichtundurchlässig, eingefärbt und vorzugsweise mit Gewebeeinlagen verstärkt sind und daß die Bodenfolie (14) und die Behälterfolie (17) der Behälterwand (4) in der Art und Qualität von Teichfolien ausgebildet sind.

26. Biofermenteranlage nach einem der Ansprüche 1 bis 25, **dadurch gekennzeichnet, daß** die Tragsäule (11) am oberen Ende mit einer Arbeitsplattform (43) versehen ist, die einen ähnlichen Umriß wie der Faulbehälter (1) aufweist und an deren Umfang die Abdeckfolie (23) der Schutzabdeckung (2) insbesonders mit einer Klemmverbindung, befestigt ist und daß die Gasbehälterfolie (23') des Fcliengasbehälters (3) gasdicht am oberen Ende der Tragsäule (11) gasdicht festgehalten ist.

27. Biofermenteranlage nach einem der Ansprüche 1 bis 26, **dadurch gekennzeichnet, daß**, insbesonders zur Arbeitsplattform (43) hin, im oberen Bereich zwischen der Abdeckfolie (23) der Schutzabdeckung (2) und der Gasbehälterfolie (23') eine Ausgleichsöffnung vorgesehen ist.

28. Biofermenteranlage nach einem der Ansprüche 1 bis 27, **dadurch gekennzeichnet, daß** die Arbeitsplattform (43) vom oberen Teil (54) der Schutzabdeckung (2), insbesonders aus einem festen Material wie Blech oder dergl., überdeckt ist, in die ein Wärmespeicher (45) insbesonders ein Warmwasserspeicher für eine thermoelektrische Wärmekraftanlage (46) oder für ein Heizgerät, insbesonders einem Warmwasserheizkessel, vorzugsweise an deren Spitze (44) eingebaut ist, wobei die Zu- und Ableitungen (47,47') für das Wärmeübertragungsmittel und/oder die elektrischen Leitungen eines Generators und/oder eine Gasableitung innen an der Tragsäule (11) nach unten und oberhalb der thermischen Abdeckung (33) an die Randeinfassung (7) des Faulbehälters (1) und vorzugsweise durch ein dort angeordnetes Mannloch (57) abgedichtet nach außen geführt oder außen an der Schutzabdeckung (2), insbesonders an einer Abspannung (48) herab, geführt sind.

29. Biofermenteranlage nach einem der Ansprüche 1 bis 28, **dadurch gekennzeichnet, daß** die Tragsäule (11) oberhalb des höchsten Standes der Gülle im Faulbehälter (1) mit einem Verbindungsflansch (49) abgeteilt ist, oberhalb von dem, vorzugsweise oberhalb der thermischen Abdeckung (33), wenigstens eine Öffnung oder eine Zuleitung, die vom oberen Ende des Foliengasbehälters (3) herabgeführt ist, für den Eintritt des Biogases angebracht ist, die in eine Waschsäule (50) mit einer Waschfüllung (52) führt, die im oberen Teil der Tragsäule (11) direkt oder indirekt eingebaut ist und am oberen Ende der Tragsäule (11) mit der Zuleitung zu der Wärmekraftanlage (46) oder dem Heizgerät oder einer Gasableitung verbunden ist.

30. Biofermenteranlage nach einem der Ansprüche 1 bis 29, **dadurch gekennzeichnet, daß** die Abspannung (48) der Schutzabdeckung (2) an der Erdoberfläche an im Boden angebrachten Verankerungen (53), die insbesonders als Bohrpfähle ausgebildet sind, befestigt ist, die an mehreren Umfangsstellen des Faulbehälters (1) und bei quadratischem Querschnitt desselben, insbesonders an dessen Ecken (54), angebracht sind.

31. Biofermenteranlage nach einem der Ansprüche 1 bis 30, **dadurch gekennzeichnet, daß** bei einer Biofermenteranlage wenigstens zwei Faulbehälter (1) mit Schutzabdeckung (2) und Foliengasbehälter (3) vorgesehen sind, die jeweils abwechselnd mit auszufaulender Flüssigkeit befüllt, ausgefault und entleert werden, wobei die Entleerung in einen weiteren, größeren Nachfaulbehälter mit Schutzabdeckung und Foliengasbehälter erfolgt, mit einem Faulraumvolumen, das dem der beiden Faulbehälter (1) zusammen, insbesonders in etwa entspricht, und aus dem die Flüssigkeit nach dem Ausfaulen abgepumpt wird.

## Claims

1. Biofermentation-Plant with at least one rotting tank for liquids to be fermentated, especially liquid manure, with roof-shaped, supported protection covering, within which is a gas proof polythene gas reservoir sheet, open towards the gas tank, overlaid with an outflow pipe and pumping device for liquid manure, with heating device and at least one tube extruder within the rotting tank and at least with one removal pipe for energy carrier, signified by having a sustaining pillar, built as tube or profiled tube (11) disposed in midst of the rottening tank (1), on it's top (18) having fixed the outer protection covering and the upper end of gasproof polythene gasreservoir sheet, and being anchored with it's lower end (19) inside the rotting tank (1), especially on the floor (8) of rotting tank,that the rotting tank (1), is shaped conic or quadratic pyramidic, tapering off from level of the upper border (6), especially from floor level (21) towards bottom surface (21), and it's tank walls (4) consist essentially of plastic sheet (14), which is laid on fleece, preferably directly on the ground (22) and a tank sheet (17), whereat tilt angle (27) of tank wall (4) to horizontal line is between 30° and 50° and preferably between 35° and 45° and that protection covering (2) of rotting tank (1), is shaped conic or quadratic pyramidic, tapering off from bottom to top, especially from ground level, from upper border of rotting tank (1), whereat gradient angle (55) is between 30° and 50° and preferably between 35° and 45°.

2. BIOFERMENTATION PLANT according Claim 1, signified, that protection cover (2) of rotting tank (1) shows gradient angle (55) of approximately between 51° and 52°.

3. BIOFERMENTATION PLANT according Claim 1 or 2, signified by, that protection covering (2) of rotting tank (1) shows a gradient angle (55) of essentially 51,87°, according tilt angle of the "Great Pyramid" covers a quadratic pyramidic rotting tank (1) tapering off to bottom with tilt angle (27) of more than 30° and preferably with at least 45°.

4. BIOFERMENTATION PLANT according Claim 1 or 3, signified by, that rotting tank wall (4) is formed by a floor film sheet (14), on which an insulating layer (15), especially PU foam, is applied throughout, on top of which, at least over a part of tank wall (4), preferably on its downer part, heating registry (16) of a heating device, especially as warm water tube registry is laid and as inside wall a caulking tank film (17) is attached.

5. BIOFERMENTATION PLANT according Claim 1, 3, or 4, signified by, that at least on one spot of rotting tank (1), if rotting tank (1) is quadratic, preferably in the middle of one of its sides, running from the upper border (6) of rotting tank (1) down to its floor (8), a concrete foundation (5) or something similar is built and closes with a horizontal floor slab (9) especially within a deepened outlet shaft (10), on which the sustaining pillar (11) is fastened with an anchoring panel or something a like.

6. BIOFERMENTATION PLANT according Claim 1 or 5, signified by, that inlet pipe (26) reaches from upper border (6) of rotting tank (1) essentially down to the floor (8) or at least to the floor region, and preferably fixed within a distance (5) from tank wall (4), especially onto the floor foundation (5), by anchor screws (24), in proof way through the tank wall by mounting profiles and in connection with a pump (39) of pumping device, a pumping chamber (20) preferably built as pumping shaft and the outlet pipe (13) ends in the horizontal floor slab (9), especially situated within a deepened outlet shaft (10) and leading along the tank wall (4) vertically up above the upper border (6) of rotting tank (1) or beneath of it through the surface soil into the pumping chamber (20).

7. BIOFERMENTATION PLANT according Claim 1, 5, or 6, signified by, that the outlet pipe (13) is also used as inlet pipe.

8. BIOFERMENTATION PLANT according Claim 1 to 7, signified by, that on the upper border (6) of rotting tank (1) a border fringe (7) is fixed, made of a concrete profile, on to which the plastic sheet (14) and the bottom tank sheet (17) of tank wall (4) and the protection film (23) of protection cover (2) and the sheeted gas reservoir (3), is fastened gas proof preferably from outside.

9. BIOFERMENTATION PLANT according Claim 1 to 7, signified by, that to the upper border (6) of rotting tank (1) with border fringe (7), a profile is attached, made of steel or alike, which consists preferably as U-Profile open to the inner side.

10. BIOFERMENTATION PLANT according Claims 1 to 9, signified by, that on sustaining pillar, at least one lateral tube extruder device (28) on the tank wall (4), especially on top of concrete foundation (5), from above, directly beneath the upper border (6) of rotting tank (1) almost down to its bottom, preferably on rolls with extruder guidance (31) in the form of runways can be moved, which is in gas proof way fastened to the tank wall (4) by anchor screws (24) over a mounting profiles (25) and a powered motor hauling device, preferably from the upper border (6) of rotting tank (1), which can be moved by a rope winch, preferably situated outside the protection cover (2).

11. BIOFERMENTATION PLANT according Claims 1 to 10, signified by, that on sustaining pillar (11) at least one middle extruder tube (29) is attached and on a extruder guidance (31), with essentially in radial direction to sustaining pillar working mixing tools (32) and that, if there are two middle extruder tubes (29, 29'), in orientation to the mixing tools (32, 32') are both sides shiftingly attached to axis of sustaining pillar (11) or that the orientation of the mixing tools partly or totally is tangentially to axis of sustaining pillar (11).

12. BIOFERMENTATION PLANT according Claims 1 to 11, signified by, that in the sustaining pillar (11), especially between flanges, a central mixing device is attached, that is essentially tube shaped and its mixing tools are horizontally directed, in axial direction especially working vertically upwards, and its motor is preferably attached axial between the flanges of sustaining pillar (11).

13. BIOFERMENTATION PLANT according Claims 1 to 12, signified by, that the sustaining pillar (11) directly or over a within led inlet tube, which is connected with a circulation pump of the pumping device, is designed for circulation of liquid manure, which, in the area of the upper border (6), especially in a distance above the maximum liquid level in rotting tank (1) can be let out preferably horizontally or slightly downwards, through at least one mouth,.

14. BIOFERMENTATION PLANT according Claim 13, signified by, that several vents, preferably nozzles, especially with different radiances and with a removal device in front the inlet of the vents, are provided.

15. BIOFERMENTATION PLANT according Claims 1 to 14, signified by, that the rotting tank (1) has a thermo covering (33) on the upper border (6), which is fixed on it and also on the sustaining pillar (11) and preferably in distance above the maximum level of manure surface in rotting tank (1) and preferably consists of the sustaining part (34) and the laid on top heat insulating component (35) and the heat insulating component preferably consists of prefabricated elements, which are tightened together by slot and key and that on the sustaining pillar additionally a heat insulating tube element (36) is placed on top the thermo covering (33).

16. BIOFERMENTATION PLANT according Claim 15, signified by, that each prefabricated element of the heat insulating element (35) of the thermo covering (33) is covered with a protection film (56,56') or that several or all prefabricated elements are covered with a upper protection film (56) and a downer protection film (56'), which are both fixed to the upper border fringe (7) and to the sustaining pillar (11).

17. BIOFERMENTATION PLANT according Claims 1 to 16, signified by, that the thermo covering (33) with the supporting part (34) and the to it attached heat insulating element (35) is laid on top the tank wall (4) at the upper rand of the rotting tank (1).

18. BIOFERMENTATION PLANT according Claims 1 to 17, signified by, that at least the bottom plastic sheet (14) and the tank sheet (17) of tank wall (4), the gas reservoir sheet (23'), of the polythene sheeted gas reservoir (3), the protection film (23) of protection covering (2) are tightened together and fixed with a single gas proof clip screwing (36), whereas the clip screwing (36) consists of a screw joint (40), which is screwed in, preferably on the surface or outer part of the profile, especially the concrete profile of border fringe (7) and a rail clip (41).

19. BIOFERMENTATION PLANT according Claim 1 or 18, signified by, that between protection film (23) and gas reservoir sheet (23'), outlet vents with small diameter are located, especially leading to the outside, so that condensed water can go out.

20. BIOFERMENTATION PLANT according Claims 1 or 18, signified by, that between the prefabricated elements of the heat insulating element (35) and the thermo covering (33) or its covering protection films and preferably to the connecting flange (49) apertures are located, leading to the polythene sheeted gas reservoir (3).

21. BIOFERMENTATION PLANT according Claims 1 to 20, signified by, that the bottom sheet (14) and the tank sheet (17) of tank wall (4), the polythene gas reservoir sheet (23') and the protection film (23') of protection cover (2) and the protection film (23) of protection covering (2) is fixed with several gas proof clamping screws (36,37,38) on to the border fringe (7) of rotting tank (1).

22. BIOFERMENTATION PLANT according Claims 1 to 21, signified by, that the tank sheet (17) and the gas reservoir sheet (23') of polythene sheeted gas reservoir (3) are together wound around a deferring slab made of foam rubber or alike (39) and together attached preferably from outside on the upper shank of a U - profile of the border fringe (7) with a rail clip (41), which is pressed down by screwing (40).

23. BIOFERMENTATION PLANT according Claims 1 to 22, signified by, that the covering protection film (23) outside and the floor plastic sheet (14) inside of U - profile of border fringe (7), are wound around a deferring wadding (39'), preferably consisting of plastic tube or something alike and fixed, each one singularly, with a rail clip (41'), which is pressed down by screwing (40).

24. BIOFERMENTATION PLANT according Claims 1 to 23, signified by, that if border fringe made as concrete profile, screwing (40)of rail clip (36) is anchored in the concrete and the edge of the concrete profile of border fringe (7) is provided with a concrete anchored edge runner (42) serving as caulking coating.

25. BIOFERMENTATION PLANT according Claims 1 to 24, signified by, that the foils, especially the protection film (23) and the foil of the gas reservoir (3), are dark, light proof, coloured and preferably strengthened with textile embedding and that the bottom plastic sheet (14) and the tank sheet (17) of the tank wall (4) are of same kind and quality as pond foils.

26. BIOFERMENTATION PLANT according Claims 1 to 25, signified by, that the sustaining pillar (11) is on the upper top equipped with a working platform (43), in a similar outline as the rotting tank (1) and on which borders the protection film (23) of protection covering (2) is fixed, especially with a rail clip and that the gas reservoir sheet (23') of polythene sheeted gas reservoir (3) is fastened gas proof on the top of sustaining pillar (11).

27. BIOFERMENTATION PLANT according Claims 1 to 26, signified by, that especially towards the working platform (43) in the upper section between protection film (23) and protection covering (2) and gas reservoir sheet (23') a compensation vent is located.

28. BIOFERMENTATION PLANT according Claims 1 to 27, signified by, that the working station (43) from the upper part of protection covering (2), especially made of strong material like sheet metal or alike, is overlaid, in which is integrated a storage water heater (45) especially a storage water heater for a thermoelectric heating plant (46) or a heating device, especially a warm water heating tank, preferably on its top (44), whereat in- and outlets (47,47') for the heating medium and/or electric mains of a generator and/or gas outlet within the sustaining pillar (11), downwards and above thermo covering (33) towards border fringe (7) of rotting tank (1) and preferably through a manhole (57) caulked up, are led outside or outside along the protection covering (2) especially along a tightrope (48).

29. BIOFERMENTATION PLANT according Claims 1 to 28, signified by, that the sustaining pillar (11) above maximum level of manure in rotting tank (1) is divided by a connecting flange, above of which, preferably above thermo covering (33) there is at least one vent or one inlet, for the entrance of the biogas, which is led down from the upper end of polythene sheeted gas reservoir (3), and which leads in a washing pillar (50) with a wash filling (52), which is directly or indirectly integrated in the upper part of sustaining pillar (11) and is connected in the upper section of sustaining pillar (11) with the supply pipe to the thermoelectric heating plant (46) or with the heating device or a gas main.

30. BIOFERMENTATION PLANT according Claims 1 to 29, signified by, that the tightrope (48) of protection covering (2) is fixed within the soil surface with soil attached anchors (53), which are especially built as drill stakes, and which are fixed on several points along the periphery of rotting tank (1) and if being of quadratic shape, especially on its edges (54).

31. BIOFERMENTATION PLANT according Claims 1 to 30, signified by, that with a biofermentation plant at least two rotting tanks (1) with protection covering (2) and sheeted gas reservoir (3) are provided, of which each alternating are filled with to be fermented liquid, left until end of fermentation process and discharged, whereas the discharging takes place into a further, bigger after fermentation container with a protection covering and gas reservoir sheet, with a rotting tank volume, which is equal to the volume of both rotting tanks (1) together, especially approximately, and from which the liquid after fermentation process is pumped out.

## Revendications

1. Usine de bio fermentation avec au moins un réservoir de décomposition pour des liquides a être fermentent, en particulier pour le lisier avec un écran de protection, une façon comme un toit qui est soutenu, dans lequel est un réservoir à gaz de feuille étanche au gaz couvre le réservoir de décomposition, le réservoir à gaz de feuille est ouvre vers la direction du réservoir de décomposition et il est équipé avec une canalisation de décharge et d'alimentation et une installation de pompage pour le lisier, avec une installation de chauffage et au moins un agitateur dans le réservoir de décomposition et fourni avec au moins une décharge pour un porteur d'énergie, alors, qui est marque qu'un pilier soutenant (11) est disposée axial dans le réservoir de décomposition de récipient (1), le pilier est forme en particulier comme un tube ou un tube profilé, au bord d'en haut du pilier (18) est fixé la recouvrement de protection extérieur (2) et le bout d'en haut du réservoir à gaz de feuille (3) est étanche preuve de gaz qui est ancrée en particulier avec la fin inférieure (19) dans le réservoir de décomposition de récipient (1), sur le sol (8) de le même, que le réservoir de décomposition de récipient (1) est formé effiler hors fonction comme une cône ou comme une pyramide carrée, du bord supérieur vers le bas trop (6), en particulier commencent de la hauteur de la surface de sol (21), et le paroi de récipient (4) est fait par une feuille de sol (14) qui est mis, de préférence directement, sur la terre (22) et une feuille de réservoir (17,qui est la paroi intérieure, auquel cas l'angle d'inclinaison (27) de la paroi de récipient (4) à la horizontale s'élève t entre 30° et 50° et de préférence environ de 35° à 45°, que l'écran de protection (2) du réservoir de décomposition de récipient (1) est formé s'effilant, comme une cône ou comme une pyramide carré, vers le haut trop, en particulier de la hauteur de la terre, commencent du bord supérieur (6) du réservoir de décomposition de récipient (1), auquel cas l'angle d'enquête (55) s'élève de préférence à plus entre 40° et 60°, et de la préférence plus de 45°.

2. Usine de bio fermentation après la revendication, marque du fait que l'écran de protection (2) de la réservoir de décomposition (1) montre un angle d'enquête (55) entre de 50° et de 55° et de préférence environ de 51° à 52°.

3. Usine de bio fermentation après la revendication 1 ou 2, marque du fait que l'écran de protection (2) du réservoir de décomposition de récipient (1) montre en conséquence un angle d'enquête (55) essentiel du 51,87°, ce qui est conforme avec l'angle d'inclinaison de la "grande pyramide" et qui couvre le réservoir de décomposition de récipient (1) effilant vers le bas, qui est formé comme une pyramide carré et qui a un angle d'inclinaison (27) de plus de 30° et de préférence au moins de 45°.

4. Usine de bio fermentation après la revendication 1, ou 3, du fait que la paroi de récipient (4) du réservoir de décomposition de récipient (1) est formée par une feuille de sol (14), sur laquelle une couche d'isolement (15) en particulier de la mousse PU, est mis sans interruption, sur laquelle, au moins sur une partie de la paroi de récipient (4), de préférence à une partie inférieure delà même, des registres de chauffage (16) d'une installation de chauffage, sont mis preuve de gaz, en particulier comme registres de canalisation de 1 'eau chaud, et comme paroi intérieure une feuille de récipient (17) qui est bien tenu.

5. Usine de bio fermentation après des revendications 1, 3 ou 4, marque du fait qu'au moins à une place du réservoir de décomposition de récipient (1), avec des grandes lignes carrées du réservoir de décomposition de récipient (1), de préférence évoluant dans le milieu d'un côté du même, du bord supérieur (6)du réservoir de décomposition de récipient (1) vers le bas jusqu'au sol (8) desquelles un béton - fondation (5) ou tels est inséré et disposée avec une galette de plancher horizontal (9), en particulier dans une cheminée approfondi de sortie, (10), isole, dont la colonne de transport (11) est attachée avec un panneau ancré (12) ou tels.

6. Usine de bio fermentation après la revendication 1 ou 5, marqué du fait que tuyau d'admission (26) à partir du bord supérieur (6) du réservoir de décomposition de récipient (1) va, essentiellement jusqu'au sol (8) ou qui descend au moins dans le secteur de sol du même et qui est fixé avec des profils de support (25), à une distance (5) de la paroi de récipient (4), en particulier sur, les boulonnages d'ancre passées (24), qui sont preuve de gaz par la paroi de récipient (4) et qui est lié à une pompe (39), à une installation de pompage dans celui, est un puits de pompe de préférence comme une chambre de pompe (20) et la canalisation de décharge (13) se déverse dans la plaque de base (9), en particulier dans un puits de sortie (10) approfondi disposé, et qui est mené de préférence sur le long de la paroi de récipient (4) vers le haut sur le bord supérieur (6) du réservoir de décomposition ou au-dessous duquel sur le sol dans une chambre de pompe (20).

7. Usine de bio fermentation après des revendications 1,5 ou 6, marqué du fait que la canalisation de décharge (13 est prévu en même temps comme tuyau d'admission.

8. Usine de bio fermentation après des revendications 1 à 7, marqué du fait que le bord supérieur (6) du réservoir de décomposition de récipient (1) est assorti avec une bande de bord (7) fait d'un profil de béton, à laquelle la feuille de sol (14) et la feuille de récipient (17) de la paroi de récipient (4) et la feuille de couverture (23) de l'écran de protection (2) et la feuille de réservoir à gaz (23 ') du réservoir à gaz de feuille (3) sont attachées preuve de gaz, de préférence de l'extérieur loin.

9. Usine de bio fermentation après une des revendications 1 à 7, marqué du fait que le bord (6) supérieur du réservoir de décomposition de récipient (1) est assorti avec une bande de bord (7) fait d'un profil, en particulier de l'acier fin ou de tels, qui se compose de préférence d'un -U- profil, ouvert vers l'intérieur.

10. Usine de bio fermentation après une des revendications 1 à 9, marqué du fait qu'au moins une extrudeuse transversale de tube (28) est arrangé à la paroi de récipient (4) en particulier à la béton fondation (5) d'en haut, directement, au-dessous du bord (6) supérieur du réservoir de décomposition de récipient (1) jusqu'au dessous presque 'à son sol (8) de préférence mobile, avec des rôles sur une conduite d'agitateur (31), avec l'aide des voies, qui est attachée par des vis de point d'attache (24) passés et obturé par la paroi de récipient (4)avec d es profils d' fixation (25) et qui est actionné de préférence avec un transport du dispositif avec un moteur, et qui est mobile avec un treuil de corde qui est placé de préférence au bord supérieur (6) du réservoir de décomposition de récipient (1), en particulier au dehors l'écran de protection (2).

11. Usine de bio fermentation après une des revendications 1 à 10, marqué du fait qu'on dispose au moins un tube moyen d'extrudeuse (29)au pilier soutenant (11),et marqué du fait qu'on dispose une conduite d'agitateur (31), en particulier avec essentiellement radialement au pilier soutenant les outils d'agitateur (32) travaillant horizontal, et qu'avec deux agitateurs de moyen (29,29) les deux sont disposés déplacé 'dans l'alignement des outils d'agitateur (32,32 ') à l'axe du pilier soutenant (11) ou que l'alignement des outils d'agitateur est prévu en partie ou complètement tangentiellement à l'axe du pilier soutenant (11).

12. Usine de bio fermentation après une des revendications 1 à 11, marqué par le fait que dans le pilier soutenant (11) particulier entre les brides, un agitateur de moyen central est installé qui est formé comme un tuyau, et que les outils de mélange duquel sont disposés horizontaux en particulier travaillant vers le haut et que le moteur de commande duquel est installé de préférence axialement entre les brides du pilier soutenant (11).

13. Usine de bio fermentation après une des revendications 1 à 12, marqué du fait que pilier soutenant (11) est prévue directement ou avec un tube de prise qui se dirige dans lequel, qui est lié à la pompe de circulation du dispositif de pompage, qui arrange en particulier la circulation du lisier, qui est placé dans la zone du bord supérieur(6),en particulier dans une distance au-dessus de l'état le plus élevé de la surface de liquide dans le réservoir de décomposition de récipient (1) par au moins une ouverture, de préférence placé horizontal ou plein au dessous.

14. Usine de bio fermentation après la revendication 13, marqué par le fait que plusieurs ouvertures, sont équipées de préférence, avec des diffuseur, en particulier avec des rayons d'action différents et avec un dispositif d'évacuation avant l'alimentation des ouvertures.

15. Usine de bio fermentation après une des revendications 1 à 14, marqué par le fait que réservoir de décomposition de récipient (1) est équipé avec une couverture (33) thermique au bord supérieur (6), qui est attachée à celui-ci et au pilier soutenant (11) et de préférence dans la distance de l'état le plus élevé de la surface de lisier dans le réservoir de décomposition de récipient (1) et qui s' est mis ensemble en particulier d'une partie porteuse (34) et d'une partie thermo isolante (35) au des sur, et la partie thermo isolante est construite de préférence des composants préfabriqués qui sont joints en particulier avec fente et clé marqué qu' un élément de tube thermo isolante (36) est placé en plus sur la couverture thermique (33) au pilier soutenant.

16. Usine de bio fermentation après la revendication 15, marqué par le fait que chaque élément préfabriqué de la partie thermo isolante (35) de la couverture thermique est couverte avec une feuille de protection (56, 56) ou que plusieurs ou tous les composants préfabriqués sont enveloppés avec une feuille de protection (56) supérieure et avec une feuille de protection inférieure (56 ') qui sont attachées toutes les deux á la bande de bord (7) et au pilier soutenant (11).

17. Usine de bio fermentation après une des revendications 1 à 16, marqué par le fait que la couverture (33) thermique est arrangé dans cette façon, que la couverture thermique se trouve au bord supérieur (6) du réservoir de décomposition de récipient (1) sur la paroi de récipient (4) avec la partie porteuse (34) et la partie thermo isolante (35) liée ou fixé.

18. Usine de bio fermentation après une des revendications 1 à 19, marqué par le fait qu'au moins la feuille de sol (14) et la feuille de récipient (17) de la paroi de récipient (4) la feuille de réservoir de gaz (23 ') du réservoir de gaz couvert par polythène (3) et la feuille de couverture (23) de l'écran de protection (2) sont attachées groupé avec une seule baise de clip (36) preuve de gaz (36), auquel cas que la baise de clip (36) se compose d'un joint de vis (40) qui de préférence est fixé à la surface supérieure ou à l'extérieur du profil, spécialement du profile de béton de la bande de bord, et 'une plaque de serrage (41).

19. Usine de bio fermentation après la revendication 1 ou 18, du fait qu'entre la feuille de couverture (23) et la feuille de réservoir à gaz (23 '), des ouvertures d'enfoncement avec de coupe transversale petit, menant en particulier à l'extérieur fabriqué de préférence par une envergure , sont fixées de telle manière que l'eau de condensation peut se retirer.

20. Usine de bio fermentation après la revendication 1 ou 19, marqué du fait qu'entre les composants préfabriqués de la partie thermo isolante (35), la couverture thermique (33) ou les feuilles de protection enveloppant de laquelle ouvertures d'enfoncement au réservoir à gaz de feuille (3) sont fixés de préférence à la bride de relation (49).

21. Usine de bio fermentation après une des revendications 1 à 20, marqué du fait que la feuille de sol (14) et la feuille de récipient (17) de la paroi de récipient (4) et la feuille de réservoir à gaz (23 ') du réservoir à gaz de feuille (3) et la feuille de couverture (23) de l'écran de protection (2) sont fixées avec plusieurs vis de retenue preuve de gaz (36,37,38) á la bande de bord (7) du réservoir de décomposition de récipient (1).

22. Usine de bio fermentation après une des revendications 1 à 21, marqué du fait que la feuille de récipient (17) et la feuille de réservoir à gaz (23 ') du réservoir à gaz de feuille (3) sont conduites en commun autour d'une insertion (39) flexible, en particulier une plaque de caoutchouc mousse ou de tels et ils sont fixés en commun de préférence au dehors sur la cuisse supérieure d'un u- profil de la bande de bord (7) avec une plaque de serrage (41) du vis de retenue maintenue par un joint de vis (40).

23. Usine de bio fermentation après une des revendications 1 à 22, marqué du fait que la feuille de couverture (23) dehors et la feuille de sol (14) à l'intérieur au u-profil de la bande de bord (7) sont conduites respectivement autour d'une insertion flexible (39) ', de préférence d'un tuyau de plastique ou tels et ils sont attachés seul avec une plaque de serrage (41) et maintenue par un joint de vis (40) respectivement particulièrement.

24. Usine de bio fermentation après un une des revendications 1 à 23, marqué du fait que lors de la formation de la bande de bord (7), le joint du vis du vis de retenue est attaché au béton avec un ancrage comme un profil de béton, et le bord du profil de béton de la bande de bord (7) est munis de coureur de bord qui est fixé au béton comme calfeutré enduire.

25. Usine de bio fermentation après une des revendications 1 à 24, est marqué par le fait que les feuilles, en particulier la feuille de couverture (23) et la feuille du réservoir de gaz polythène (3) sont colorées opaque, et sombre et elles sont renforcées de préférence avec des insertions de tissu et que la feuille de sol (14) et la feuille de récipient (17) de la paroi de récipient (4) sont formées dans la sorte et la qualité des feuilles piscicoles.

26. Usine de bio fermentation après une des revendications 1 à 25, marqué du fait que le pilier soutenant (11) est équipé à la fin supérieure d'une plate-forme de travail (43) qui montre des grandes lignes semblables comme le réservoir de décomposition de récipient (1) et à l'ampleur de laquelle la feuille de couverture (23) de l'écran de protection (2) est attachée avec une relation de serrage et que la feuille de réservoir à gaz (23) du réservoir à gaz de feuille (3) est preuve au gaz à la fin supérieure du pilier soutenant (11).

27. Usine de bio fermentation après une des revendications 1 à 26, marqué du fait que, en particulier vers la plate-forme de travail (43), dans le secteur supérieur entre la feuille de couverture (23) de l'écran de protection (2) et de la feuille de réservoir à gaz (23 ') une ouverture de compensation est prévue.

28. Usine de bio fermentation après une des revendications 1 à 27, marqué par le fait que la plate-forme de travail (43) de la partie supérieure (54) de l'écran de protection (2), est couverte en particulier d'un matériel ferme comme de la tôle ou tels, dans lesquelles un régénérateur (45) en particulier une mémoire d'eau chaude pour une usine de chauffage thermoélectrique (46) ou pour un réchauffeur, en particulier à une chaudière d'eau chaude est inséré de préférence, au dessus de laquelle (44), auquel cas les dans et sorties ( 47,47') pour les moyens de transmission de chaleur et/ ou les câbles électriques d'un dynamo et/ ou une sortir de gaz sont conduits à l'extérieur à l'intérieure du pilier soutenant vers le bas et au-dessus de la couverture thermique (33) jusqu'a la bande de bord (7) du réservoir de décomposition de récipient (1) et obturé de préférence par un trou de homme (57) disposé ici ou dehors à l'écran de protection (2), en particulier à une élimination (48) vers le bas.

29. Usine de bio fermentation près une des revendications 1 à 28, marqué, du fait que le pilier soutenant (11) est partagé au-dessus de l'élevé état du lisier dans le réservoir de décomposition (1) avec un bride (49) lié, au-dessus de celui, de préférence au-dessus le thermique couvrir (33) conduit, au moins un ouvrir ou une adduction, qui est conduit du supérieur fin du réservoir à gaz (3) et l'ouvrer et l'adduction sont fixées pour 1 entrée 'du biogaz, qui conduit dans un pilier de lavage (50) avec un remplissage de lavage (52), qui est inséré dans la partie supérieur du pilier soutenant (11) direct ou indirect et qui est liés au supérieur fin du pilier soutenant (11) avec une pipe d'approvisionnement jusque a l'usine de chauffage thermoélectrique (46) ou le réchauffeur ou un sortir de gaz.

30. Usine de bio fermentation après une des revendications 1 à 29, puisque marqué que l'élimination (48) de l'écran de protection (2) est attachée à la surface de la terre aux points d'attache (53) qui sont en particulier formées comme pieux de perçage, fixés dans le sol, qui sont fixés à plusieurs postes d'ampleur du réservoir de décomposition de récipient (1) et avec une coupe transversale carrée de le même, en particulier à leurs coins (54).

31. Usine de bio fermentation après une des revendications 1 à 30, marqué du fait que chez une usine de bio fermentation, au moins deux réservoir de décomposition de récipient (1) avec l'écran de protection (2) et le réservoir à gaz de feuille (3) sont prévus , qui sont vidés alternant avec des liquides particulièrement engrais, laisser ici jusqu'au fin de la procédé de fermentation et après vidage qui arrive dans un autre plus grand réservoir de décomposition avec de l'écran de protection (2) du réservoir à gaz de feuille, avec un volume de secteur qui correspond ensemble, en particulier plus ou moins à cela des deux réservoir de décomposition de récipient (1), dont le liquide est évacué après le fin de la procédé de fermentation.
